(19) Europäisches Patentamt
European Patent Office
Office européen des brevets

(11) **EP 1 234 845 B1**

(12) **EUROPEAN PATENT SPECIFICATION**

(45) Date of publication and mention
of the grant of the patent:
**12.11.2008 Bulletin 2008/46**

(51) Int Cl.:
*C08G 64/30* (2006.01)     *C07C 68/02* (2006.01)
*C07C 68/08* (2006.01)     *C07C 69/96* (2006.01)

(21) Application number: **02251209.9**

(22) Date of filing: **22.02.2002**

(54) **Process for producing diphenyl carbonate and process for producing aromatic polycarbonates**

Verfahren zur Herstellung von Diphenylcarbonat und Verfahren zur Herstellung von Polycarbonaten

Procédé de préparation de carbonate de diphényle et procédé de préparation de polycarbonates

(84) Designated Contracting States:
**BE**

(30) Priority: **26.02.2001 JP 2001049617**
**21.05.2001 JP 2001150441**

(43) Date of publication of application:
**28.08.2002 Bulletin 2002/35**

(73) Proprietor: **Mitsubishi Chemical Corporation**
**Minato-ku**
**Tokyo 108-0014 (JP)**

(72) Inventors:
• **Miyamoto, Masaaki,**
**c/o Mitsubishi Chemical Corp.**
**Kitakyushu-shi,**
**Fukuoka-ken 806-0004 (JP)**
• **Kuma, Kiyoji,**
**c/o Mitsubishi Chemical Corporation**
**Kitakyushu-shi,**
**Fukuoka-ken 806-0004 (JP)**
• **Hyoudou, Narutoshi,**
**c/o Mitsubishi Chemical Corp.**
**Kitakyushu-shi,**
**Fukuoka-ken 806-0004 (JP)**

• **Fujimoto, Eiji,**
**c/o Mitsubishi Chemical Corp.**
**Kitakyushu-shi,**
**Fukuoka-ken 806-0004 (JP)**
• **Urashima, Hidetoshi,**
**c/o Mitsubishi Chemical Corp.**
**Kitakyushu-shi,**
**Fukuoka-ken 806-0004 (JP)**

(74) Representative: **Woods, Geoffrey Corlett**
**J.A. KEMP & CO.**
**14 South Square**
**Gray's Inn**
**London**
**WC1R 5JJ (GB)**

(56) References cited:
**EP-A- 0 722 931          GB-A- 1 096 936**
**GB-A- 1 517 423**

• **PATENT ABSTRACTS OF JAPAN vol. 016, no. 342 (C-0966), 24 July 1992 (1992-07-24) & JP 04 100824 A (ASAHI CHEM IND CO LTD), 2 April 1992 (1992-04-02)**

**Description**

The present invention relates to a process for producing diphenyl carbonate, more particularly to a process for obtaining purified diphenyl carbonate with a low chlorine content by means of distillation purification. The purified diphenyl carbonate obtained according to the present invention is useful as a starting material for the production of aromatic polycarbonates by molten ester exchange method. The produced aromatic polycarbonates are useful as a resin material for optical disc substrates, transport containers for precision electronic parts such as IC and LED, housings of electronic devices, and the like.

Various methods are known for the preparation of diphenyl carbonate, for example, a method in which phenol is reacted with phosgene in the presence of a quaternary ammonium salt as a catalyst, and a method which comprises reacting an aqueous solution of a metallic phenoxide with phosgene in the presence of an organic solvent. The commercially produced diphenyl carbonate contains various kinds of contaminants no matter which method is used, so that purification is essential for practical use of this material. The contaminants include organic and inorganic chlorine compounds, metallic ions, iron compounds, reaction intermediates such as chlorophenyl formate, and organic solvents used in the production processes. Among these contaminants, the organic and inorganic chlorine compounds give a serious influence to the polymerization rate and hue of the product in the production of aromatic polycarbonates, so that the purification step for removing such contaminants is especially important. (See Japanese Patent Publication (KOKOKU) Nos. 38-1373, 7-96613 and 6-18868, and Japanese Patent Application Laid-Open (KOKAI) Nos. 63-97627 and 64-24829).

Japanese Patent Application Laid-Open (KOKAI) No. 4-100824 discloses a process for producing an aromatic polycarbonate by reacting an aromatic dihydroxyl compound with diaryl carbonate, in which (1) no polymerization catalyst is used, and (2) the diaryl carbonate used is one having a chlorine content of not more than 0.05 ppm and a xanthone content of not more than 10 ppm. It is stated that the "chlorine" referred to therein means one which exists as an acid or a salt such as hydrochloric acid, sodium chloride, potassium chloride, etc., or one existing in the organic compounds such as phenyl chloroformate, and that such chlorine can be determined from the measurement of chlorine ions by potentiometric titration using a silver nitrate solution.

For obtaining such diphenyl carbonate with a low chlorine content, various purification methods have been proposed, such as: Urea is added to diphenyl carbonate and the mixture is melted by heating (Japanese Patent Publication (KOKOKU) No. 42-9820); Molten diphenyl carbonate is washed with water and then distilled (Japanese Patent Application Laid-Open (KOKAI) No. 7-138208); Diphenyl carbonate is fractionated from a melt of crude diphenyl carbonate and crystallized (Japanese Patent Application Laid-Open (KOKAI) No. 8-3119); Diphenyl carbonate is distilled in the presence of a basic substance (Japanese Patent Application Laid-Open (KOKAI) No. 8-198816).

As viewed above, various methods have been proposed for reducing chlorine contained in trace amounts in the products. However, according to the method in which molten diphenyl carbonate is washed with water and then distilled, although it is indeed possible to reduce the easily extractable chlorine of diphenyl carbonate to the order of several ten ppb, this method is ineffective for reducing the volatile chlorine produced by the decomposition of the organic chlorine compounds existing as impurities in the product. Thus, even if the easily extractable chlorine can be reduced by washing with water, there takes place decomposition of the organic chlorine compounds in the next distillation step to cause contamination with volatile chlorine, raising the chlorine content in dipheny carbonate to several ten ppb or above. Obviously, such diphenyl carbonate is unsuited for use as a starting material for the production of aromatic polycarbonates free of tinting.

Further, in the case of the method in which diphenyl carbonate is distilled in the presence of a basic substance, the basic substance used there needs to be one with a fairly high concentration, and especially when such a basic substance is of the type which is insoluble in diphenyl carbonate, it is hardly possible to reduce the content of trace chlorine in diphenyl carbonate obtained from the distillation column top even if the basic substance is added to a high concentration. This is considered ascribable to the fact that since the basic substance is insoluble, no effect thereof is produced in the portion of diphenyl carbonate where the basic substance is not in contact, and consequently diphenyl carbonate is distilled out without being reduced in chlorine content. Further, it was found that if a basic substance of high concentration is left under a high temperature at the bottom of the distillation column, a phenolate of the basic substance is produced, and this phenolate is causative of pitting corrosion of the austenitic stainless steel used as the column material.

Also, another starting material phosgene needs to be high in purity for obtaining diphenyl carbonate of high purity. Phosgene has been generally produced from carbon monoxide and chlorine by using active carbon as catalyst, but this method had the various problems such as mentioned below:

(1) In case where commercial active carbon is used as the catalyst, the produced phosgene is contaminated with large quantities of the by-product impurities, especially carbon tetrachloride, due to the impurities present in active carbon (Japanese Patent Publication (KOKOKU) No. 6-29129) or because of rise of reaction temperature by rapid progress of the reaction (Japanese Patent Publication (KOKOKU) No. 55-14044).

(2) It is considered that metallic impurities contained in commercial active carbon encourage the formation of byproducts such as carbon tetrachloride in the reaction of carbon monoxide and chlorine. It is deemed expedient to keep the content of metallic impurities of active carbon below a specific level by pickling or other means (Japanese Patent Publication (KOKOKU) No. 6-29129), but actually it is troublesome and unpractical to conduct pickling of large quantities of active carbon, and it is also difficult to efficiently remove the metallic impurities by such means.

(3) In order to avoid rise of reaction temperature, it has been proposed to pass stock gas along the surface alone of the catalyst layer or to provide cooling tubes in multiple stages. However, the former method involves the problem that volumetric efficiency of the reactor drops excessively, while the latter measure has the concern that the reactor structure would be complicated.

(4) It has been also proposed to cool the catalyst layer externally, but in this case, too, carbon tetrachloride is formed in large quantities because the central portion of the catalyst layer becomes high in temperature. (Kirk, et al: Encyclopedia of Chemical Technology, 2nd Ed., Vol. 5, etc.)

When phosgene containing impurities such as carbon tetrachloride is used for the preparation of diphenyl carbonate, the organic chlorine impurities are formed as byproducts, and it is difficult to reduce the chlorine content in the produced diphenyl carbonate. Diphenyl carbonate with a high chlorine content causes a drop of activity or deterioration of hue in the polymerization reaction by ester exchange with a hydroxyl compound, so that it is unsuited for use as a starting material for the production of polycarbonates. Recently, therefore, it is tried to liquefy phosgene gas to remove low-boiling point impurities in phosgene, or to distill phosgene to remove high-boiling point impurities, or to purify liquefied phosgene with an adsorbent such as active carbon. According to any of these methods, however, the amount of harmful phosgene present in the plant increases, giving rise to the concern over its environmental effect in the even of accident. Also, since equipment for phosgene purification is required, construction and service costs increase.

The object of the present invention is to provide a process for producing diphenyl carbonate, which realizes continuous industrial production of this material and features effective removal of impurities stemming from chlorine present in diphenyl carbonate which cause tinting or become an impediment to polymerization in production of aromatic polycarbonates by molten ester exchange, such removal of impurities being carried out while controlling decomposition of diphenyl carbonate.

As a result of the present inventors' earnest studies to solve the above problems, it has been found that in the process for purifying crude diphenyl carbonate containing water and impurities by continuous distillation, it is possible to phenomenally lessen the amount of basic substance required for reduction of chlorine and, as a result, it becomes possible to minimize damage to the distillation column material, by incorporating first-stage distillation for removing impurities having a lower boiling point than diphenyl carbonate by bringing this material into countercurrent contact with a basic substance, as it is possible in this first-stage distillation to let an aqueous solution of the basic substance contact efficiently with chlorine. It has been further found that in the second-stage distillation for recovering purified diphenyl carbonate or the recovery distillation for collecting and distilling the bottom product, it is possible to minimize damage to the column material by continuously extracting a determined amount of the bottom product from the bottom of the distillation column to avoid long-time residence of the material under a high temperature and avoiding formation of phenolate of the basic substance as much as possible. By taking these means, it has become possible to efficiently neutralize the acidic substance formed as a by-product in the column without affecting the column material while inhibiting diphenyl carbonate from being hydrolyzed, and to reduce the chlorine-derived acidic polymerization inhibitory substances to a total chlorine of not more than 20 ppb, thereby enabling obtainment of purified diphenyl carbonate useful as a starting material for the production of polycarbonates with little or no tinting.

The present invention has been attained on the basis of the above findings.

Thus, in the first aspect of the present invention, there is provided a process for producing diphenyl carbonate which comprises: (a) preparing crude diphenyl carbonate by reacting phosgene and phenol; and (b) continuous distillation of the crude diphenyl carbonate which continuous distillation comprises a first-stage distillation for removing water and impurities having a lower boiling point than diphenyl carbonate, and a second-stage distillation for recovering purified diphenyl carbonate;

a basic substance being brought into countercurrent contact with said low-boiling point impurities in said first-stage distillation.

Also, it has been found that in the preparation of diphenyl carbonate by reacting phosgene and phenol, the above-described diphenyl carbonate purification method is expedient when using phosgene which has not been subjected to liquefaction purification. Since an effect of no liquefaction purification of phosgene, it has become possible to provide a safe and uncostly process in which the amount of phosgene present in the plant is minimized in the reaction step for forming crude diphenyl carbonate. It has been further found that by the introduction of the said purification step in which a basic substance and low-boiling point impurities are brought into countercurrent contact with each other, it is possible to positively lessen the chlorine-originating unnecessary components even in the diphenyl carbonate obtained by using non-liquefaction-purified phosgene.

Thus, in another aspect of the present invention, there is provided a process as described above in which in the step (a) gaseous phosgene containing not less than 0.1 vol% of impurities and obtained by reacting carbon monoxide and chlorine in the presence of a catalyst is used without liquefaction purification.

In still another aspect, the present invention provides a process for producing an aromatic polycarbonate, which comprises (a) producing diphenyl carbonate by the above-described process and (b) reacting the diphenyl carbonate with an aromatic dihydroxyl compound.

The present invention is described in further detail below.

1. Preparation of crude diphenyl carbonate

The diphenyl carbonate purification technique of the present invention can be applied for purification of crude diphenyl carbonate containing water and impurities obtained by the various known methods (Japanese Patent Publication (KOKOKU) Nos. 38-1373 and 58-50977, Japanese Patent Application Laid-Open (KOKAI) Nos. 7-53473 and 7-53474, etc.). This technique is especially effective for purifying the product obtained by reacting phenol and phosgene in the presence of a quaternary ammonium salt, aromatic heterocyclic nitrogen-containing basic compound or a salt thereof as the catalyst, contacting the reaction mixture with an alkaline solution to neutralize the mixture, separating the organic phase from the aqueous phase, washing it with water, and again separating the organic phase from the aqueous phase. Particularly, it is possible with this technique to effectively remove the residue of the basic catalyst used for the reaction from diphenyl carbonate.

As the basic catalyst used for the above reaction, there can be used quaternary ammonium salts including organic and inorganic acid salts such as tetramethylammonium and tetraethylammonium; aromatic heterocyclic nitrogen-containing basic compounds such as pyridine, quinoline, picoline, imidazoles, benzimidazole, pyrazoles, triazoles and benztriazoles; and their salts including organic and inorganic acid salts of the said aromatic heterocyclic nitrogen-containing basic compounds. All of these basic catalysts have a lower boiling point than diphenyl carbonate.

The reaction is carried out, for instance, by heating a mixture of phenol and a quaternary ammonium salt, an aromatic heterocyclic nitrogen-containing basic compound or a salt thereof to 120 to 190 °C, and introducing gaseous phosgene into the mixture with sufficient stirring. As for the amount of phosgene to be introduced, in the case of phosgene, it is preferably introduced in an amount of not more than 1.0 mole, more preferably 0.4 to 0.5 mole, based on 1,0 mole of phenol. The catalyst is used preferably in an amount of 0.1 to 10% by mole, more preferably 0.5 to 5% by mole based on the aromatic monohydroxyl compound which is a base material. In the mixture after the reaction, there are contained diphenyl carbonate, unreacted phenol, hydrochloride of quaternary ammonium salt or aromatic heterocyclic nitrogen-containing basic compound used as catalyst, and other trace impurities. The content of chlorine (easily extractable chlorine) ranges from 300 to 60,000 ppm depending on the amount of the catalyst used. Next, the reaction mixture is brought into contact with an alkaline solution of 70 to 95 °C for neutralizing the mixture, then the organic phase is separated from the aqueous phase in the pH range of 7 to 13, the separated organic phase is further contacted with hot water of 70 to 95 °C, and then the organic phase is again separated from the aqueous phase. The hydrochloride type basic catalyst is converted into a free type by the neutralization, with the best part thereof being transformed into the organic phase. Therefore, in the crude diphenyl carbonate separated as the organic phase, there are usually contained several ten to several hundred ppb of easily extractable chlorine and about 10,000 ppm of water.

In the preparation of crude diphenyl carbonate by reacting phosgene with phenol, it is preferable to use phosgene which has not been subjected to liquefaction purification. In the following, preparation of crude diphenyl carbonate using such non-liquefaction-purified phosgene is explained.

As phosgene, there was used one obtained by passing a mixed gas of carbon monoxide and chlorine through a packed bed of active carbon for reacting them by the conventional method.

According to this method, the highest temperature portion of the packed bed reaches 300 to 400 °C due to the reaction heat. Since usually external cooling is conducted with cooling water, the gas temperature at the exit of the reactor is around 100 °C. The ratios of carbon monoxide and chlorine offered to the reaction are adjusted so that carbon monoxide will be slightly in excess of the stoichiometrical amount. In the formed phosgene gas are contained carbon monoxide, carbon dioxide, carbon tetrachloride and chlorine as impurities, but the main constituent is carbon monoxide. (Although hydrogen chloride is also contained, this is not included in the impurities.).

Generally, the impurity content, especially carbon monoxide content rises at the start or shutdown of the phosgene producing apparatus. This is for the reason that at the start, chlorine is supplied gradually corresponding to heat generation while passing carbon monoxide through the packed bed of active carbon, and at the time of shutdown, chlorine supply is first stopped, and after phosgene in the active carbon packed bed has been purged out by carbon monoxide, supply of carbon monoxide is stopped. Phosgene with a high content of such impurities is also usable in the present invention. If desired, formed phosgene may be treated by a pretreatment column until the phosgene production restores the stationary state, and the production of diphenyl carbonate may be resumed after the phosgene quality is stabilized.

In the present invention, the produced phosgene is directly offered to use for the production of diphenyl carbonate without undergoing liquefaction purification. This makes it possible to integrate the production of phosgene and the production of diphenyl carbonate, and to minimize the amount of phosgene retained in the production plant.

The reaction is carried out, for instance, by heating a mixture of phenol and an aromatic nitrogen-containing heterocyclic compound or a salt thereof used as a catalyst to 120 to 190 °C, and introducing phosgene gas into this mixture with sufficient stirring. The amount of phosgene introduced is preferably not more than 1.0 mole, more preferably 0.4 to 0.5 mole based on one mole of phenol. The reaction conditions are set so that not less than 70% by weight, preferably not less than 80% by weight of the impurities in phosgene will be discharged out of the reaction system together with the by-product hydrogen chloride.

In order to accelerate gas effluence, the reactor atmosphere is brought into a slightly pressed state, that is, the internal pressure of the reactor is preferably adjusted to stay in the range of 0.10 to 0.15 MPa in consideration of possible pressure loss in the discharge gas treating system so that while phosgene remains dissolved in the reaction solution, the by-product hydrogen chloride will be quickly led out of the reactor together with the impurities. The effluent gas is guided into a condenser where phenol, carried by the said gas is condensed, while the noncondensed gas containing impurities is subjected to a pretreatment and then released into the atmosphere. The pretreatment includes neutralization of the acidic substances in a pretreatment column where an alkaline solution, such as a sodium hydroxide solution, is circulated, or neutralization of residual gas after recovering water-washed hydrogen chloride as hydrochloric acid. The residual gas after the neutralization treatment is released into the atmosphere in the form as it is, or subjected to an incineration treatment. Also, hydrogen chloride may be oxidized with oxygen at a high temperature in the presence of a catalyst to generate chlorine from hydrogen chloride, and the generated chlorine may be liquefied and recovered.

On the other hand, the reaction solution contains diphenyl carbonate, unreacted phenol, trace impurities and hydrochloride of the aromatic nitrogen-containing heterocyclic compound used as catalyst. Easily extractable chlorine is also contained in an amount of 300 to 60,000 ppm depending on the amount of the catalyst used. Usually this reaction solution is subjected to a two-stage washing treatment, that is, the said solution is first washed with an alkaline solution to neutralize the hydrochloride of the aromatic nitrogen-containing heterocyclic compound, producing a free heterocyclic compound and alkali chloride, and then the product is washed with water to remove alkali chloride. For example, the reaction solution is brought into contact with a 70 to 95 °C alkaline solution for neutralization, then the organic phase is separated from the aqueous phase in the pH range of 7 to 13, the separated organic phase being contacted with hot water of 70 to 95 °C, and then the organic phase is again separated from the aqueous phase. As a result of the above treatment, the concentration of easily extractable chlorine in the organic phase stays somewhere between several ten and several hundred ppb while the water content registers approximately 10,000 ppm.

The diphenyl carbonate purification step according to the present invention comprises first-stage distillation and second-stage distillation described below.

First-stage distillation is designed to remove impurities having a lower boiling point than diphenyl carbonate, such as water, catalyst and unreacted phenol in crude diphenyl carbonate. By bringing the low-boiling point impurities into countercurrent contact with the basic substance, it is possible to almost perfectly neutralize the acidic substances generated by heating during distillation. Also, by continuously and efficiently extracting the bottom product from the column bottom, which is conducted for the purposes of controlling hydrolysis of diphenyl carbonate in the second-stage distillation and avoiding corrosion by the basic substances, it is possible to remove the chlorine-derived impurities and to efficiently recover purified diphenyl carbonate with a low chlorine content.

2. First-stage distillation

In the present invention, crude diphenyl carbonate in the organic phase is continuously distilled in a distillation column, and austenitic stainless steel is selected as the composing material of this distillation column in consideration of its pitting corrosion resistance and other advantageous properties.

In the first-stage distillation, first of all, the impurities having a lower boiling point than diphenyl carbonate, such as water, free type basic catalyst and unreacted phenol are removed. Further, in this first-stage distillation, a basic substance is supplied, and it is brought into countercurrent contact with the low-boiling point impurities. The basic substance is usually supplied in the form of a solution. As its supply site, a position where the basic substance and the low-boiling point impurities can be brought into gas/liquid countercurrent contact with each other in the column is selected. Any position may be selected if it is located upwards of the bottom product discharge port at the bottom of the distillation column. However, it is more expedient to supply the basic substance from an upper position of the column where the polar molecules are concentrated, because this has the effect of promoting dispersion of the basic substance and dissociation of the basic catalyst. It is also effective to previously mix the basic substance in the crude diphenyl carbonate containing water and impurities and supply the mixture as a distillation solution from a middle position of the column. By taking such a position for the supply of the basic substance, it is possible to enhance dispersibility of the basic substance during first-stage distillation and promote thermal decomposition of the organic chlorine compounds. As a result, hydrochloric acid formed by the decomposition can be fixated as a metallic salt having substantially no vapor pressure, with no need of combining it with a free type basic catalyst which is to be recovered for reuse. Also, the chlorine content (total chlorine) in the bottom product in the second-stage distillation can be reduced to 20 ppb or below, preferably 10 ppb or below.

As the basic substance supplied for first-stage distillation, it is possible to use any basic substance which is capable of fixating hydrochloric acid formed by thermal decomposition of an organic chlorine compound as a metallic salt having substantially no vapor pressure, but it is preferable to use a basic substance which comprises at least one of alkaline metals, alkaline earth metals and their oxides, hydroxides and carbonates. The typical examples of these substances are alkaline metals: sodium, potassium and lithium; alkaline earth metals: magnesium and barium; oxides of alkaline metals or alkaline earth metals: magnesium oxide and calcium oxide; hydroxides of alkaline metals or alkaline earth metals: sodium hydroxide and potassium hydroxide; carbonates of alkaline metals or alkaline earth metals: lithium carbonate, magnesium carbonate, calcium carbonate and barium carbonate. Among these substances, strongly basic substances are preferred in view of efficiency, and specifically sodium hydroxide, potassium hydroxide are preferred as they need to be added only in small quantities.

The amount of the basic substance to be supplied should be decided in consideration of the amount of total chlorine in the crude diphenyl carbonate supplied to first-stage distillation. Excessive supply of the basic substance increases the decomposition loss of diphenyl carbonate and is also undesirable from the viewpoint of corrosion, while scanty supply of the basic substance can not provide a satisfactory chlorine reducing effect. Therefore, a basic substance is usually used in an amount which is 0.5 to 20 times, preferably 1 to 10 times the said amount of total chlorine by mole.

First-stage distillation in the present invention is not subject to any specific restrictions except that it should be continuous distillation. Usually a distillation (rectification) system incorporating a refluxing device is used, but it is also possible to adopt single distillation. The degree of vacuum for the distillation may be optionally selected, but in view of the boiling point of diphenyl carbonate, it will be appropriate to carry out distillation under a degree of vacuum of 0.1 to 100 kPa. The water content in the bottom product in the first-stage distillation is usually reduced to less than 100 ppm.

3. Second-stage distillation

In the second-stage distillation, purified diphenyl carbonate is recovered from the bottom product of the first-stage distillation. In this operation, preferably the bottom product of the second-stage distillation is continuously extracted, and the bottom residence time ($\theta t$) is adjusted to be within 5 hours, more preferably within 2 hours. Here, "bottom residence time" is the residence time of the bottom product at the column bottom, and is decided by the bottom capacity in relation to the flow rate of the bottom product.

The bottom product of the first-stage distillation, which contains substantially no water (less than 100 ppm), still maintains alkaline metal or alkaline earth metal salts which have fixated hydrochloric acid in the first-stage distillation, but it does not give any adverse effect to the distillation column material as far as it is not converted to phenolate. It is considered, however, that once the bottom product is converted to phenolate, the basic substance is brought into a state of being dissolved in molten diphenyl carbonate and becomes strongly acidic, provoking corrosion of austenitic stainless steel in an increasing tempo. Therefore, it is important not only to lessen as much as possible the amount of the basic substance supplied to the first-stage distillation but to also continuously and efficiently extract the bottom product from the column bottom at a constant rate by suitable means such as a metering pump and regulator valves so that the basic substance will not stay in the column for a long time.

The concentration rate in the second-stage distillation is preferably as high as it can be from the viewpoint of recovery of diphenyl carbonate, but usually it is selected from 2 to 1,000 times, preferably 5 to 200 times. On the other hand, the yield of the alkaline metal phenolate is a function of reaction temperature, time and concentration, so that these parameters are preferably kept small for controlling the reaction. Regarding the temperature, however, although it depends on the balance with pressure because of the distillation operation, it is impossible to select a temperature lower than the melting point of diphenyl carbonate, and usually a temperature of 100 to 300 °C is preferred. Practically, therefore, it is the best available means to shorten the residence time in the distillation column and to lower the concentration.

4. Recovery distillation

The bottom product of the second-stage distillation can be purified by additional distillation for the purpose of further recovering the residual diphenyl carbonate from the bottom product. In this case, it is expedient to continuously extract the bottom product of the recovery distillation and to adjust the bottom residence time ($\theta t$) to be within 10 hours, especially within 5 hours.

Also, for the purification of the distillate diphenyl carbonate, it may be recycled back to the step of neutralization and washing of the hydrochloride type basic catalyst used for the preparation of crude diphenyl carbonate.

5. Production of aromatic polycarbonate

Diphenyl carbonate obtained from the above-described method is subjected to ester exchange reaction with a dihydroxyl compound and used as a starting material for the production of an aromatic polycarbonate of a specified molecular weight. The amount of diphenyl carbonate supplied is usually 1.01 to 1.30, preferably 1.02 to 1.20 in molar ratio to the dihydroxyl compound.

When producing an aromatic polycarbonate by ester exchange method, usually an ester exchange catalyst is used. In the present invention, mainly alkaline metal compounds and/or alkaline earth metal compounds are used as ester exchange catalyst, and basic compounds such as basic boron compounds, basic phosphorus compounds, basic ammonium compounds and amine-based compounds may be used in combination. These catalysts may be used either singly or as a mixture of two or more.

Catalyst is used in an amount of $1 \times 10^{-9}$ to $1 \times 10^{-3}$ moles based on one mole of dihydroxyl compound, but in case of using an alkaline metal compound and/or an alkaline earth metal compound having advantageous properties and good handling qualities as catalyst, usually, it is used in an amount of $1 \times 10^{-8}$ to $1 \times 10^{-5}$ moles, preferably $2 \times 10^{-8}$ to $8 \times 10^{-6}$ moles based on one mole of dihydroxyl compound. If the catalyst amount is below the above-defined range, there may not be obtained sufficient polymerization activity for producing a desired hydroxyl-terminated polycarbonate having a specified molecular weight, and if the catalyst amount exceeds the above range, the produced polymer may be deteriorated in its hue and tends to have many branches of the molecule.

Examples of the alkaline metal compounds usable as catalyst in the present invention include sodium hydroxide, potassium hydroxide, lithium hydroxide, cesium hydroxide, sodium hydrogencarbonate, potassium hydrogencarbonate, lithium hydrogencarbonate, cesium hydrogencarbonate, sodium carbonate, potassium carbonate, lithium carbonate, cesium carbonate, sodium acetate, potassium acetate, lithium acetate, cesium acetate, sodium stearate, potassium stearate, lithium stearate, cesium stearate, sodium borohydride, potassium borohydride, lithium borohydride, cesium borohydride, sodium tetraphenylborate, potassium tetraphenylborate, lithium tetraphenylborate, cesium tetraphenylborate, sodium benzoate, potassium benzoate, lithium benzoate, cesium benzoate, disodium hydrogenphosphate, dipotassium hydrogenphosphate, dicesium hydrogenphosphate, disodium phenylphosphate, dipotassium phenylphosphate, dilithium phenylphosphate, dicesium phenylphosphate, alcoholates and phenolates of sodium, potassium and cesium, and disodium salts, dipotassium salts, dilithium salts and dicesium salts of bisphenol A.

Examples of the alkaline earth metal compounds include calcium hydroxide, barium hydroxide, magnesium hydroxide, strontium hydroxide, calcium hydrogencarbonate, barium hydrogencarbonate, magnesium hydrogencarbonate, strontium hydrogencarbonate, calcium carbonate, barium carbonate, magnesium carbonate, strontium carbonate, calcium acetate, barium acetate, magnesium acetate, strontium acetate, calcium stearate, barium stearate, magnesium stearate, and strontium stearate.

Examples of the basic boron compounds include tetramethylboron, tetraethylboron, tetrapropylboron, tetrabutylboron, trimethylboron, trimethylbenzylboron, trimethylphenylboron, triethylmethylboron, triethylbenzylboron, triethylphenylboron, tributylbenzylboron, tributylphenylboron, tetraphenylboron, benzyltriphenylboron, methyltriphenylboron, and butyltriphenylboron.

Examples of the basic phosphorus compounds include triethylphosphine, tri-n-propylphosphine, triisopropylphosphine, tri-n-butylphosphine, triphenylphosphine, tributylphosphine, and quaternary phosphonium salts.

Examples of the basic ammonium compounds include tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylphenylammonium hydroxide, tributylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide.

Examples of the amine-based compounds include 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline.

Ester exchange reaction is usually carried out in two or more stages. The first-stage reaction is usually conducted under reduced pressure at 120 to 260°C, preferably 180 to 240°C, for 0.1 to 5 hours, preferably 0.1 to 3 hours. Then the reaction temperature is raised while reducing the degree of vacuum of the reaction system to carry out polycondensation reaction while distilling away the by-product phenol finally under a vacuum of usually not higher than 1.33 kPa (10 mmHg) at usually 240 to 330 °C.

The reaction formula may be batch, continuous, or a combination thereof. The reaction apparatus may be tank type, tube type or tower type.

The chlorine content shown in the present specification was determined in the following way.

Easily extractable chlorine:

Diphenyl carbonate (5 g) was dissolved in purified toluene (10 ml) by heating, then ultra-pure water (10 ml) was added, and the mixture was stirred (by a magnetic stirrer at 1,000 rpm) at room temperature for 10 minutes. Chlorine in the aqueous phase was quantitatively analyzed by ion chromatography to determine its amount, which was expressed by weight ratio to diphenyl carbonate and indicated as easily extractable chlorine.

Total chlorine:

Diphenyl carbonate (5 g) and pyridine (0.1 g), which is a trapping agent of volatile chlorine, were filled in a glass tube, and after sealing the tube in vacuo under cooling, it was immersed in an oil bath and heated at 190 °C for 4 hours. After cooled to room temperature, the sealed glass tube was cracked and the content chlorine was determined in the same way as in the case of the easily extractable chlorine described above, and its amount was expressed by weight ratio to diphenyl carbonate and indicated as total chlorine.

The reaction solution composition and gas composition were analyzed by gas chromatography, while water, pyridine and phenol were determined by Karl Fischer's water content analyzer and high performance liquid chromatography, respectively. Alkaline metal salt concentration was determined by atomic-absorption spectroscopy after ashing diphenyl carbonate and dissolving it in a strong acid.

According to the present invention, it is possible to continuously reduce the chlorine-derived acidic polymerization inhibitory substances in diphenyl carbonate to a total chlorine of less than 20 ppb by controlling decomposition of diphenyl carbonate, so that the product of the present invention is useful for the industrial production of diphenyl carbonate as a starting material for polycarbonates with little or no tinting.

EXAMPLES

The present invention is further explained by the following examples, which examples are however merely intended to be illustrative and not limitative to the scope of the invention.

Preparation Example 1 for crude diphenyl carbonate

Two sets of jacketed, glass-lined reactor (inner capacity: 60 litres; provided with an overflow tube at the position of 29 L liquid level) connected to an external heater of oil circulation system were provided continuously in connection to each other. A condenser-adapted gas discharge pipe for discharging the produced hydrochloric acid gas out of the system was joined to the second reactor. The molten phenol, to which 5 mol% of pyridine had been added and stirred, was supplied continuously into the first reactor at a rate of about 29 1/hr (equivalent to 29.7 kg/hr of phenol and 1.24 kg/hr of pyridine) and heated to 150 °C. polyfluoroethylene-lined disc turbine blades were used as agitator, and phosgene with a molar ratio of 0.46 to the phenyl supplied was continuously supplied (14.4 kg/hr) to the first reactor.

The effluent reaction mixture from the first reactor was supplied to the second reactor via an overflow pipe, and the reaction mixture effusing from the second reactor was led into a degassing column and brought into countercurrent contact with nitrogen gas (0.5 m$^3$/hr into the reaction mixture).

The reaction mixture (composition: 88 wt% diphenyl carbonate, 7 wt% phenol and 5 wt% hydrochloride of pyridine) flowing out from the degassing column was led into a jacketed, polyfluoroethylene-lined neutralization mixing tank connected to an external heater of a hot water circulation system, and was mixed and contacted with approximately 5% by weight of an aqueous sodium hydroxide solution at 80 °C for 10 minutes to make the pH 8.5. After neutralization, the organic phase was left in a settler for 30 minutes, then separated from the aqueous phase and transferred into a rinsing and mixing tank. In the rinsing and mixing tank, the organic phase was mixed with hot water of about 30% by weight based on the organic phase for 10 minutes and then left in the settler for 30 minutes. The aqueous phase was separated to obtain crude diphenyl carbonate (water content, 10,000 ppm; pyridine, 3.0 wt%; phenol, 7.0 wt%). The easily extractable chlorine in the produced crude diphenyl carbonate was 43 ppb and the total chlorine was 3,800 ppb. The diphenyl carbonate obtained here is called "crude diphenyl carbonate 1".

Preparation Example 2 for crude diphenyl carbonate

(1) Preparation of phosgene

Carbon monoxide gas (carbon monoxide: 98.1 vol%; hydrogen: 1.9 vol%; carbon dioxide: trace; water: trace) was supplied into a phosgene reactor via a gas mixer at a rate of 3.56 Nm$^3$/hr, then chlorine gas (chlorine: 99.8 volume % and oxygen: 0.2 volume %) was supplied into the gas mixer gradually in accordance with the reactor temperature and mixed with carbon monoxide gas, and the mixture was led into the phosgene reactor. After the reactor temperature has been stabilized two hours later, chlorine was supplied constantly at a rate of 3.36 Nm$^3$/hr. The reactor comprised a column (100 mm$\phi$ × 2,500 mmH) packed with granular active carbon, and was cooled by cooling water. Reaction pressure was set at 3.3 kg/cm$^2$G, and the generated phosgene gas was discharged out of the reactor at 70 °C. The composition of crude phosgene gas in the normal operation was 92.3 vol% phosgene, 3.7 vol% carbon monoxide, 3.9 vol% hydrogen chloride, trace hydrogen, 0.1 vol% carbon dioxide, 100 vol ppm carbon tetrachloride, and trace chlorine, and its yield was 3.63 Nm$^3$/hr. The best part of this crude phosgene gas was supplied to the diphenyl carbonate reactor, and a slight amount of surplus was released into the atmosphere after it was made innoxious in a pretreatment column where a sodium hydroxide solution was circulated.

(2) Preparation of diphenyl carbonate

50 °C molten phenol and a catalyst pyridine were supplied continuously at the rates of 30.0 kg/hr (0.319 kmol/hr) and 1.26 kg/hr (0.0159 kmol/hr), respectively, into a first reactor under sufficient agitation. Then the said crude phosgene was supplied continuously into the first reactor at a rate of 3.56 Nm$^3$/hr (phosgene: 14.5 kg/hr). The first reactor was provided with an overflow pipe at the position of internal volume of 30 litres, and the reaction mixture was passed into a second reactor under sufficient agitation via an overflow pipe as a gas/liquid mixed phase. The second reactor was also provided with an overflow pipe at the position of internal volume of 30 litres. Temperature was maintained at 150 °C in both reactors. The reaction solution running out of the second reactor was supplied into a degassing column. For completing the reaction of phenyl chloroformate and phenol which are the intermediates in the degassing column, 160 °C nitrogen gas was brought into countercurrent contact with the reaction solution at a rate of 300 NL/hr to urge exodus of chlorine gas from the reaction solution. The discharge gas from the degassing column was joined with the discharge gas from the second reactor, passed through a condenser and made innoxious in a pretreatment column. The impurities in crude phosgene were almost entirely fluxed into the discharge gas. In the stationary state, the reaction solution was obtained at a flow rate of 35.7 kg/hr from the degassing column, and its composition comprised 88.1% by weight of diphenyl carbonate. The supplied phosgene was converted almost 100% to diphenyl carbonate.

The effluent reaction solution from the degassing column was passed into a jacketed Teflon-lined neutralizing tank where the reaction solution was mixed with about 5% by weight of an aqueous sodium hydroxide solution at 80 °C for 10 minutes, and the mixture was led into and left in a settler for 30 minutes and separated into an aqueous phase and an organic phase. Neutralization was conducted to make the pH of the aqueous phase 8.5. The organic phase was transferred into a water washing tank where about 30% by weight of hot water was added to the organic phase and mixed for 10 minutes, and the mixture was guided into and left in a settler for 30 minutes. The aqueous phase was separated to obtain crude diphenyl carbonate (water content: 1.0 wt%; pyridine: 3.0 wt%; phenyl: 7.0 wt%). The easily extractable chlorine in this crude diphenyl carbonate was 43 ppb and the total chlorine was 5,000 ppb. The diphenyl carbonate obtained here is called "crude diphenyl carbonate 2".

Example 1

The crude diphenyl carbonate 1 and a 0.1N sodium hydroxide solution were supplied continuously into a middle portion of a first-stage distillation column at the rates of about 28 kg/hr and 70 mL/hr, respectively. Used as the first-stage distillation column was a SUS 316-made continuous distillation column with a theoretical plate number of 8. This distillation column measured 150 mm in inner diameter and 3.5 m in height, and was provided with a refluxing device at an upper part and a stock solution feed section at the middle, and the concentrating and recovering sections were filled with Sulzer Packing (mfd. by Sumitomo Heavy Machinery Industries Co., Ltd.). Distillation was carried out under the following conditions: degree of vacuum = 2.7 kPa; heating medium oil temperature = about 220 °C; top temperature = 80-100 °C ; reflux ratio = 1; distillation rate = about 12%. From the column top, water, free pyridine and unreacted phenol, which are the substances having a lower boiling point than diphenyl carbonate, were removed as distillates. From the column bottom, the bottom product was extracted at a rate of 24.5 kg/hr, with a bottom residence time of 20 minutes. The water content in the extracted bottom product diphenyl carbonate was below the limit of detection (10 ppm), and the pyridine and phenol contents were below the limit of detection (1 ppm) and 50 ppm, respectively. The easily extractable chlorine in the bottom product was 2,300 ppb, which was almost equal to the total chlorine of 2,310 ppb determined by a heat test. This is for the reason that almost whole of the organic chlorine compound present in the crude diphenyl carbonate was decomposed as a result of countercurrent contact with sodium hydroxide in the first-stage distillation column. The metallic Na concentration in the bottom product was 6.5 ppm, which indicates that the whole amount of Na in the supplied sodium hydroxide solution was balanced to the bottom side.

The bottom product of the first-stage distillation column was then continuously supplied into a second-stage distillation column. Used as the second-stage distillation column was a SUS 304-made continuous distillation column with a theoretical plate number of 8. This distillation column measured 200 mm in inner diameter and 3.5 m in height,

and was provided with a refluxing device at an upper part and a stock solution feed section at the middle, and the concentrating and recovering sections were filled with Sulzer Packing (mfd. by Sumitomo Heavy Machinery Industries Co., Ltd.). Distillation was carried out under the following conditions: degree of vacuum = 2.7 kPa; heating medium oil temperature = 240 °C ; top temperature = about 180 °C ; reflux ratio = 0.5; distillation rate = about 90%. From the column top, purified diphenyl carbonate was obtained as distillate at a rate of about 22 kg/hr, and from the column bottom, the bottom product was extracted at a rate of 2.5 kg/hr, with a bottom residence time of one hour. Phenol in the top product (purified diphenyl carbonate) was 80 ppm, and the easily extractable chlorine and total chlorine were both not more than 4 ppb, indicating high purity of the product. On the other hand, the easily extractable chlorine and total chlorine in the bottom product were both 23,000 ppb, and the metallic Na concentration was 64 ppm. Thus, chlorine and Na were concentrated in conformity to the distillation rate at the bottom, and it was found that chlorine in the bottom product of the said first-stage distillation column has been fixated wholly as NaCl.

Example 2

The same procedure as defined in Example 1 was conducted except that the bottom product extracted from the bottom of the second-stage distillation column was subjected to continuous recovery distillation under the conditions described below, and that the diphenyl carbonate recovered from the top of the recovery distillation column was recycled to the neutralization mixing tank in the above Preparation Example 1 for crude diphenyl carbonate. The operation was carried out continuously for about one month.

Used for the diphenyl carbonate recovery distillation was a SUS 316-made continuous distillation column with a theoretical plate number of 8, which measured 100 mm in inner diameter and 2.5 m in height and was provided with a refluxing device at an upper part and a stock solution feed section at the middle, with its concentrating and recovering sections being packed with Sulzer Packing (mfd. by Sumitomo Heavy Machinery Industries Co., Ltd.). The operating conditions of this recovery distillation column were adjusted as follows: degree of vacuum = 2.7 kPa ; heating medium oil temperature = about 240 °C ; top temperature = 180 °C ; reflux ratio = 0.5; bottom residence time = 2 hours; continuous extraction of bottom product = 0.3 kg/hr.

As a result, high-purity diphenyl carbonate with a chlorine content of not more than 4 ppb and a phenol content of 70 ppm could be obtained continuously at a rate of about 22 kg/hr as the distillate from the top of the second-stage distillation column. One month after the operation, each of the distillation columns (first-stage distillation column, second-stage distillation column and recovery distillation column) was opened and its inside was checked. No trace of corrosion was detected.

Comparative Example 1

Continuous distillation for purification was carried out in the same way as in Example 1 except that no sodium hydroxide solution was supplied to the crude diphenyl carbonate 1. The easily extractable chlorine and total chlorine in the purified diphenyl carbonate produced as distillate from the second-stage distillation were 50 ppb and 90 ppb, respectively.

Comparative Example 2

2 kg of the said crude diphenyl carbonate 1 and 5 mL of 0.1N sodium hydroxide solution were supplied to the still portion of a distillation column packed with Sulzer Labo Packing (mfd. by Sumitomo Heavy Machinery Industries Co., Ltd.) with a theoretical plate number of 8, and subjected to batch distillation for purification. Distillation was carried out under the conditions of: degree of vacuum = 2.7 kPa; reflux ratio = 1; distillation rate = 400 g/hr, and the temperature of the heating medium was raised gradually to distill away the low-boiling point impurities. When the column top temperature reached 180 °C which is the boiling point of diphenyl carbonate, the distillate diphenyl carbonate was batched off upon every production of 300 g and fractionated to 5 cuts. In each cut, the easily extractable chlorine was 50 to 70 ppb, and the total chlorine detected after the heat test was several hundred ppb. This poor result is attributable to slow decomposition of the organic chlorine compounds and insufficient neutralization of the formed volatile chlorine because of insufficient dispersion of sodium hydroxide which was merely allowed to exist at the bottom.

Comparative Example 3

Batch distillation was carried out according to the procedure of Comparative Example 2 except that the 0.1N sodium hydroxide solution was supplied in an amount of 20 mL. The easily extractable chlorine and total chlorine in the distillate diphenyl carbonate were 30 ppb and 55 ppb, respectively.

Comparative Example 4

Continuous distillation was carried out according to the procedure of Example 1 except that the 0.1N sodium hydroxide solution was supplied to the bottom of the first-stage distillation column at a rate of 70 mL/hr. The easily extractable chlorine and total chlorine in the purified diphenyl carbonate distillate obtained from the second-stage distillation were 35 ppb and 50 ppb, respectively.

Comparative Example 5

Long-time continuous distillation was carried out according to the procedure of Example 1 except that the 0.1N sodium hydroxide solution was supplied to the bottom of the first-stage distillation column at a rate of 300 mL/hr. The easily extractable chlorine and total chlorine in the purified diphenyl carbonate obtained from the second-stage distillation were both 25 ppb, and the phenol concentration was 720 ppm, which was rather high. The amount of phenol distilled away in the first-stage distillation was also large, resulting in accelerated decomposition of diphenyl carbonate during distillation. Further, in one month after start of the operation, pitting corrosion was seen at several locations in the still (made of SUS 304) of the second-stage distillation column.

The reaction conditions in Examples 1 and 2 and Comparative Examples 1 to 5 are shown in Table 1.

Table 1

| | Example 1 | Example 2 |
|---|---|---|
| Type of distillation | Continuous | Continuous (1 month) |
| Supply of crude DPC | 28kg/hr | 28kg/hr |
| 0.1N-NaOHaq | 70mL/hr | 70mL/hr |
| (Supply of NaOH) | 0.28g/hr | 0.28g/hr |
| (NaOH/crude DPC) | 10ppm | 10ppm |
| Alkali feed position | Middle of first-stage column | Middle of first-stage column |
| Distillation rate | | |
| First-stage distillation | 12% | 12% |
| Second-stage distillation | 90% | 90% |
| Recovery distillation | | 90% |
| φt | | |
| First-stage distillation | 20 min | 20 min |
| Second-stage distillation | 1 hr | 1 hr |
| Recovery distillation | | 2 hr |
| First-stage distillation | | |
| Bottom product | | |
| $H_2O$ concentration | <10ppm | |
| Pyridine concentration | <1ppm | |
| Phenol concentration | 50ppm | |
| Easily extractable chlorine | 2300ppb | |
| Total chlorine | 2310ppb | |
| Na concentration | 6.5ppm | |
| Second-stage distillation | | |
| Distillate | | |
| Phenol concentration | 80ppm | 70ppm |
| Easily extractable chlorine | <4ppb | <4ppb |
| Total chlorine | <4ppb | <4ppb |
| Bottom product | | |
| Easily extractable chlorine | 23000ppb | |
| Total chlorine | 23000ppb | |
| Na concentration | 64ppm | |
| Condition of corrosion | | No corrosion |

## Table 1 (Continued)

| | Comp. Example 1 | Comp. Example 2 |
|---|---|---|
| Type of distillation | Continuous | Batch |
| Supply of crude DPC | 28kg/hr | 2kg |
| 0.1N-NaOHaq | None | 5mL |
| (Supply of NaOH) | None | 0.02g |
| (NaOH/crude DPC) | None | 10ppm |
| Alkali feed position | None | Still |
| Distillation rate | | |
|   First-stage distillation | 12% | |
|   Second-stage distillation | 90% | |
|   Recovery distillation | | |
| φt | | |
|   First-stage distillation | 20 min | |
|   Second-stage distillation | 1 hr | |
|   Recovery distillation | | |
| First-stage distillation | | |
|   Bottom product | | |
|    $H_2O$ concentration | | |
|    Pyridine concentration | | |
|    Phenol concentration | | |
|    Easily extractable chlorine | | |
|    Total chlorine | | |
|    Na concentration | | |
| Second-stage distillation | | |
|   Distillate | | |
|    Phenol concentration | | |
|    Easily extractable chlorine | 50ppb | 50-70ppb |
|    Total chlorine | 90ppb | Few hundred ppb |
|   Bottom product | | |
|    Easily extractable chlorine | | |
|    Total chlorine | | |
|    Na concentration | | |
| Condition of corrosion | | |

## Table 1 (Continued)

| | Comp. Example 3 | Comp. Example 4 |
|---|---|---|
| Type of distillation | Batch | Continuous |
| Supply of crude DPC | 2kg/hr | 28kg |
| 0.1N-NaOHaq | 20ml | 70mL |
| (Supply of NaOH) | 0.08g | 0.28g |
| (NaOH/crude DPC) | 40ppm | 10ppm |
| Alkali feed position | Still | Bottom of first-stage column |
| Distillation rate | | |
| First-stage distillation | | 12% |
| Second-stage distillation | | 90% |
| Recovery distillation | | |
| $\phi$t | | |
| First-stage distillation | | 20 min |
| Second-stage distillation | | 1 hr |
| Recovery distillation | | |
| First-stage distillation | | |
| Bottom product | | |
| $H_2O$ concentration | | |
| Pyridine concentration | | |
| Phenol concentration | | |
| Easily extractable chlorine | | |
| Total chlorine | | |
| Na concentration | | |
| Second-stage distillation | | |
| Distillate | | |
| Phenol concentration | | |
| Easily extractable chlorine | 30ppb | 35ppb |
| Total chlorine | 55ppb | 50ppb |
| Bottom product | | |
| Easily extractable chlorine | | |
| Total chlorine | | |
| Na concentration | | |
| Condition of corrosion | | |

14

## Table 1 (Continued)

|  | Comp. Example 5 |
|---|---|
| Type of distillation | Continuous (1 month) |
| Supply of crude DPC | 28kg |
| 0.1N-NaOHaq | 300mL |
| (Supply of NaOH) | 1.2g |
| (NaOH/crude DPC) | 43ppm |
| Alkali feed position | Bottom of first-stage column |
| Distillation rate |  |
| First-stage distillation | 12% |
| Second-stage distillation | 90% |
| Recovery distillation |  |
| φt |  |
| First-stage distillation | 20 min |
| Second-stage distillation | 1 hr |
| Recovery distillation |  |
| First-stage distillation |  |
| Bottom product |  |
| $H_2O$ concentration |  |
| Pyridine concentration |  |
| Phenol concentration |  |
| Easily extractable chlorine |  |
| Total chlorine |  |
| Na concentration |  |
| Second-stage distillation |  |
| Distillate |  |
| Phenol concentration | 720ppm |
| Easily extractable chlorine | 25ppb |
| Total chlorine | 25ppb |
| Bottom product |  |
| Easily extractable chlorine |  |
| Total chlorine |  |
| Na concentration |  |
| Condition of corrosion | Pitting corrosion in still of second-stage column |

Example 3

The said crude diphenyl carbonate and a 0.1N sodium hydroxide solution were supplied continuously into the middle section of the first-stage distillation column at the rates of about 28 kg/hr and 70 mL/hr, respectively. Used as the first-stage distillation column was a 150 mm ID, 3.5 m high continuous distillation column with a theoretical plate number of 8, which was provided with a refluxing device at an upper part and a stock feed section at the middle, with its concentrating and recovering sections being packed with Sulzer Packing (mfd. by Sumitomo Heavy Machinery Industries Co., Ltd.). Distillation was carried out under the conditions of: pressure = 2.7 kPa; reboiler heating medium temperature = 220 °C; column top temperature = 80-100°C; reflux ratio = 1; distillation rate = about 12%, to remove water, free pyridine and unreacted phenol which are the substances having a lower boiling point than diphenyl carbonate. The bottom product was extracted from the column bottom at a rate of 24.5 kg/hr. The solution residence time at the bottom was 20 minutes. The water content in the extracted diphenyl carbonate was undetected (less than 10 ppm), and the pyridine and phenol contents were undetected (less than 1 ppm) and 50 ppm, respectively. The easily extractable chlorine was 2,700 ppb, almost equal to the total chlorine which was 2,710 ppb. These results can be accounted for by the fact that substantially the whole amount of organic chlorine compounds in diphenyl carbonate was decomposed by contact with sodium hydroxide in the first-stage distillation column. The sodium concentration in the bottom product was 6.5 ppm, indicating that sodium in the supplied sodium hydroxide solution has been transferred wholly into the bottom product.

Then the bottom product was supplied continuously to the second-stage distillation column. Used as the second-stage distillation column was a 200 mm ID, 3.5 m high continuous distillation column, with a theoretical plate number of 8, which was provided with a refluxing device at an upper part and a stock feed section at the middle, with its concentrating and recovering sections being packed with Sulzer Packing. Distillation was carried out under the following conditions: pressure = 2.7 kPa; reboiler heating medium temperature = about 240°C; column top temperature = about 180 °C; reflux ratio = 0.5; distillation rate = about 90%. Purified diphenyl carbonate was obtained from the column top at a rate of about 22 kg/hr while the bottom product was extracted from the column bottom at a rate of 2.5 kg/hr. The solution residence time at the column bottom was one hour. Phenol in the purified diphenyl carbonate was 80 ppm, and the easily extractable chlorine and total chlorine were both less than 4 ppb, ensuring obtainment of a high-purity product. On the other hand, the easily extractable chlorine and total chlorine in the bottom product were both 27,000 ppb and the sodium concentration was 64 ppm. Chlorine and sodium were concentrated in the bottom product in accordance with the distillation rate, and it was found that chlorine in diphenyl carbonate obtained from the first-stage distillation column was fixated wholly as sodium chloride.

Example 4

188.5 g (0.880 mole) of diphenyl carbonate obtained in Example 2, 182.6 g (0.800 mole) of bisphenol A (produced by Shin Nittetsu Chemical Co., Ltd.) and 50 $\mu$l (0.35 $\mu$mole/mole of bisphenol A) of a 0.18 wt% cesium carbonate solution as ester exchange catalyst were supplied into a 500 ml glass flask equipped with a stirrer and a distilling device, and after replacing the inside atmosphere of the reactor with nitrogen gas, the contained materials were dissolved at 210 °C under a nitrogen gas atmosphere. After the materials were dissolved perfectly, this condition was maintained under normal pressure for one hour. The pressure in the reactor was lowered gradually to 13 kPa, to distill out phenol, and this condition was maintained for one hour. Thereafter, the polymerization temperature was raised to 240 °C while the pressure in the reactor was reduced gradually to 2.0 kPa, and after conducting polymerization for one hour, the temperature was further raised to 270 °C and the pressure in the reactor was reduced to 67 Pa to continue polymerization for one hour. A rise of viscosity was confirmed during the above operation, and after recovering the product, its molecular weight and hue were evaluated by the methods described below. A polycarbonate with Mv = 17,300 and YI = 1.4 was obtained.

Molecular weight (Mv):

Specific viscosity ($\eta$sp) of a methylene chloride solution having a polycarbonate concentration (C) of 0.6 g/dl was measured at 20°C by a Ubbellohde viscometer, and the molecular weight was determined from this specific viscosity using the following equations:

$$\eta sp/C = [\eta]\ (1 + 0.28\ \eta sp)$$

$$[\eta] = 1.23 \times 10^{-4}\ (Mv)^{0.83}$$

Hue:

A 10% polycarbonate solution in methylene chloride solution was put into a 25 mm-diameter, 55 mm high glass cell, and the tristimulus values X, Y and Z, which are the absolute values of color, were determined by a color tester (SC-1-CH mfd. by Suga Testing Machines Co., Ltd.). Then the YI value, which is an index of the degree of yellowness, was calculated from the following equation:

$$YI = 100/Y \times (1.28X - 1.06Z)$$

Example 5

The same procedure as defined in Example 4 was conducted except for use of the diphenyl carbonate obtained in Example 3 to obtain a polycarbonate with Mv = 17,300 and YI = 1.4.

**Claims**

1. A process for producing diphenyl carbonate which comprises: (a) preparing crude diphenyl carbonate by reacting phosgene and phenol; and (b) continuous distillation of the crude diphenyl carbonate, which continuous distillation comprises a first-stage distillation for removing water and impurities having a lower boiling point than diphenyl carbonate, and a second-stage distillation for recovering purified diphenyl carbonate;
a basic substance being brought into countercurrent contact with said low-boiling point impurities in said first-stage distillation.

2. A process according to claim 1, wherein in the second-stage distillation, a bottom product is extracted continuously and the bottom residence time is within 5 hours.

3. A process according to claim 2 further comprising recovery distillation for further recovering diphenyl carbonate from said bottom product, in which the bottom product is extracted continuously and the bottom residence time is within 10 hours.

4. A process according to any one of claims 1 to 3, wherein the basic substance comprises at least one of alkaline metals, alkaline earth metals and their oxides, hydroxides and carbonates.

5. A process according to claim 4, wherein the basic substance comprises sodium hydroxide and/or potassium hydroxide.

6. A process according to any one of claims 1 to 5, wherein the chlorine content (total chlorine) in the purified diphenyl carbonate is not more than 20 ppb.

7. A process according to any one of claims 1 to 6, wherein distillation is carried out in a column comprising austenitic stainless steel.

8. A process according to any one of claims 1 to 7, wherein step (a) comprises reacting phosgene and phenol in the presence of a quaternary ammonium salt, an aromatic heterocyclic nitrogen-containing basic compound or a salt thereof, bringing the produced reaction mixture into contact with an alkaline solution to neutralize the mixture, separating an organic phase from an aqueous phase, washing the separated organic phase with water, and again separating it from an aqueous phase.

9. A process according to claim 8 and to claim 3, wherein the distillate of the bottom product recovered continuously by the recovery distillation is recycled to the neutralization washing step in which said reaction mixture is brought into contact with an alkaline solution.

10. A process according to any one of claims 1 to 9, wherein, in step (a), gaseous phosgene containing not less than 0.1 vol% of impurities and obtained by reacting carbon monoxide and chlorine in the presence of a catalyst is used without liquefaction purification.

**11.** A process according to claim 10, wherein not less than 70% by weight of impurities carried into the reaction zone with the phosgene are discharged in a gaseous state together with by-product hydrogen chloride from the reaction zone during the reaction.

**12.** A process according to claim 10 or 11, wherein the phosgene contains one or more of carbon monoxide, carbon dioxide, carbon tetrachloride and chlorine as impurities, with carbon monoxide constituting at least the main component of the impurities.

**13.** A process for producing an aromatic polycarbonate which comprises (a) producing diphenyl carbonate by a process according to any one of claims 1 to 12 and (b) reacting the diphenyl carbonate with an aromatic dihydroxyl compound.

**Patentansprüche**

**1.** Verfahren zur Herstellung von Diphenylcarbonat, das folgendes umfaßt: (a) Herstellung von rohem Diphenylcarbonat durch Umsetzung von Phosgen und Phenol und (b) eine kontinuierliche Destillation des rohen Diphenylcarbonats, wobei die kontinuierliche Destillation eine erste Destillation zur Entfernung von Wasser und Vereinreinigungen mit einem niedrigeren Siedepunkt als Diphenylcarbonat und eine zweite Destillation zur Rückgewinnung von gereinigtem Diphenylcarbonat umfaßt; wobei eine basische Substanz in Gegenstromkontakt mit den Verunreinigungen mit niedrigem Siedepunkt in der ersten Destillation gebracht wird.

**2.** Verfahren gemäß Anspruch 1, worin in der zweiten Destillation ein Sumpfprodukt kontinuierlich extrahiert wird und die Sumpfverweilzeit innerhalb von 5 Stunden liegt.

**3.** Verfahren gemäß Anspruch 2, das ferner eine Rückgewinnungsdestillation zur weiteren Rückgewinnung von Diphenylcarbonat aus dem Sumpfprodukt umfaßt, in der das Sumpfprodukt kontinuierlich extrahiert wird und die Sumpfverweilzeit innerhalb von 10 Stunden liegt.

**4.** Verfahren gemäß mindestens einem der Ansprüche 1 bis 3, worin die basische Substanz mindestens einen Vertreter von Alkalimetallen, Erdalkalimetallen und ihren Oxiden, Hydroxiden und Carbonaten umfaßt.

**5.** Verfahren gemäß Anspruch 4, worin die basische Substanz Natriumhydroxid und/oder Kaliumhydroxid umfaßt.

**6.** Verfahren gemäß mindestens einem der Ansprüche 1 bis 5, worin der Chlorgehalt (gesamtes Chlor) in dem gereinigten Diphenylcarbonat nicht mehr als 20 ppb beträgt.

**7.** Verfahren gemäß mindestens einem der Ansprüche 1 bis 6, worin die Destillation in einer Säule durchgeführt wird, die austenitischen nichtrostenden Stahl umfaßt.

**8.** Verfahren gemäß mindestens einem der Ansprüche 1 bis 7, worin Schritt (a) die Umsetzung von Phosgen und Phenol in Gegenwart eines quaternären Ammoniumsalzes, einer aromatischen heterocyclischen stickstoffhaltigen basischen Verbindung oder eines Salzes davon, das Inkontaktbringen der erzeugten Reaktionsmischung mit einer alkalischen Lösung zur Neutralisation der Mischung, das Abtrennen der organischen Phase von der wäßrigen Phase, das Waschen der abgetrennten organischen Phase mit Wasser und das nochmalige Abtrennen dieser von einer wäßrigen Phase umfaßt.

**9.** Verfahren gemäß Anspruch 8 und Anspruch 3, worin das Destillat des Sumpfprodukts, das durch die Rückgewinnungsdestillation kontinuierlich rückgewonnen wurde, zum Neutralisationswaschschritt zurückgeführt wird, in dem die Reaktionsmischung mit einer alkalischen Lösung in Kontakt gebracht wird.

**10.** Verfahren gemäß mindestens einem der Ansprüche 1 bis 9, worin in Schritt (a) gasförmiges Phosgen, das nicht weniger als 0,1 Vol.% Verunreinigungen enthält und durch Umsetzung von Kohlenmonoxid und Chlor in Gegenwart eines Katalysators erhalten wird, ohne Kondensationsreinigung verwendet wird.

**11.** Verfahren gemäß Anspruch 10, worin nicht weniger als 70 Gew.% der Verunreinigungen, die mit dem Phosgen der Reaktionszone zugeführt wurden, in einem gasförmigen Zustand zusammen mit dem Nebenprodukt Chlorwasserstoff während der Reaktion aus der Reaktionszone ausgestoßen werden.

**12.** Verfahren gemäß Anspruch 10 oder 11, worin das Phosgen mindestens einen oder mehrere Vertreter von Kohlenmonoxid, Kohlendioxid, Tetrachlorkohlenstoff und Chlor als Verunreinigungen enthält, wobei Kohlenmonoxid zumindest die Hauptkomponente der Verunreinigungen bildet.

**13.** Verfahren zur Herstellung eines aromatischen Polycarbonats, das (a) die Herstellung von Diphenylcarbonat durch ein Verfahren gemäß mindestens einem der Ansprüche 1 bis 12 und (b) die Umsetzung von Diphenylcarbonat mit einer aromatischen Dihydroxylverbindung umfaßt.


**Revendications**

**1.** Procédé pour la production de carbonate de diphényle, qui comprend : (a) la préparation de carbonate de diphényle brut en faisant réagir du phosgène et du phénol ; et (b) la distillation continue du carbonate de diphényle brut, cette distillation continue comprenant une première étape de distillation pour éliminer l'eau et les impuretés ayant un point d'ébullition inférieur à celui du carbonate de diphényle, et une deuxième étape de distillation pour la récupération du carbonate de diphényle purifié ;
une substance basique étant mise en contact, à contre-courant, avec lesdites impuretés à bas point d'ébullition dans ladite première étape de distillation.

**2.** Procédé selon la revendication 1, dans lequel, dans la deuxième étape de distillation, on extrait, de façon continue, un produit résiduel, et le temps de séjour au fond est de moins de 5 heures.

**3.** Procédé selon la revendication 2, comprenant, en outre, une distillation de récupération pour récupérer davantage de carbonate de diphényle à partir du dit produit résiduel, dans lequel le produit résiduel est extrait de façon continue, et le temps de séjour au fond est de moins de 10 heures.

**4.** Procédé selon une quelconque des revendications 1 à 3, dans lequel la substance basique comprend au moins un composant choisi parmi les métaux alcalins, les métaux alcalinoterreux et leurs oxydes, hydroxydes et carbonates.

**5.** Procédé selon la revendication 4, dans lequel la substance basique comprend de l'hydroxyde de sodium et/ou de l'hydroxyde de potassium.

**6.** Procédé selon le quelconque des revendications 1 à 5, dans lequel la teneur en chlore (chlore total) dans le carbonate de diphényle purifié n'est pas supérieure à 20 ppb.

**7.** Procédé selon l'une quelconque des revendications 1 à 6, dans lequel la distillation est effectuée dans une colonne comprenant de l'acier inoxydable austénitique.

**8.** Procédé selon le quelconque des revendications 1 à 7, dans lequel l'étape (a) comprend la réaction de phosgène et de phénol en présence d'un sel d'ammonium quaternaire, d'un composé basique azoté hétérocyclique aromatique ou d'un sel de celui-ci, la mise en contact du mélange réactionnel produit avec une solution alcaline pour neutraliser le mélange, la séparation d'une phase organique à partir d'une phase aqueuse, le lavage de la phase organique séparée avec de l'eau, et, à nouveau, sa séparation à partir d'une phase aqueuse.

**9.** Procédé selon la revendication 8 et la revendication 3, dans lequel le distillat du produit résiduel récupéré de façon continue par la distillation de récupération, est recyclé à l'étape de lavage et de neutralisation dans laquelle ledit mélange réactionnel est mis en contact avec une solution alcaline.

**10.** Procédé selon l'une quelconque des revendications 1 à 9, dans lequel, dans l'étape (a), on emploie, sans purification par liquéfaction, du phosgène gazeux contenant pas moins de 0,1 % en volume d'impuretés, et obtenu en faisant réagir du monoxyde de carbone et du chlore en présence d'un catalyseur.

**11.** Procédé selon la revendication 10, dans lequel pas moins de 70 % en poids des impuretés transportées dans la zone de réaction avec le phosgène sont évacués dans un état gazeux ensemble avec le chlorure d'hydrogène formé en tant que sous-produit, à partir de la zone de réaction au cours de la réaction.

**12.** Procédé selon la revendication 10 ou 11, dans lequel le phosgène contient une ou plusieurs substances choisies parmi le monoxyde de carbone, le dioxyde de carbone, le tétrachlorure de carbone et le chlore en tant qu'impuretés, le monoxyde de carbone constituant au moins le principal composant des impuretés.

13. Procédé pour la production d'un polycarbonate aromatique, qui comprend (a) la production de carbonate de diphényle par un procédé selon l'une quelconque des revendications 1 à 12, et (b) la réaction du carbonate de diphényle avec un composé aromatique dihydroxylé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 381373 A
- JP 7096613 A
- JP 6018868 A
- JP 63097627 A
- JP 6424829 B
- JP 4100824 A
- JP 429820 A
- JP 7138208 A

- JP 8003119 A
- JP 8198816 A
- JP 6029129 A
- JP 55014044 A
- JP 58050977 A
- JP 7053473 A
- JP 7053474 A

**Non-patent literature cited in the description**

- **KIRK et al.** Encyclopedia of Chemical Technology. vol. 5